Europäisches Patentamt

European Patent Office

Office européen des brevets

(19)

(11) Veröffentlichungsnummer: **0 286 100 B1**

(12) **EUROPÄISCHE PATENTSCHRIFT**

(45) Veröffentlichungstag der Patentschrift: **16.10.91**

(51) Int. Cl.⁵: **C12P 5/02**

(21) Anmeldenummer: **88105567.7**

(22) Anmeldetag: **07.04.88**

(54) **Verfahren und Vorrichtung zur Aufbereitung und anaeroben Vergärung biogen-organischer Abfälle.**

(30) Priorität: **08.04.87 DE 3711813**

(43) Veröffentlichungstag der Anmeldung:
**12.10.88 Patentblatt 88/41**

(45) Bekanntmachung des Hinweises auf die
Patenterteilung:
**16.10.91 Patentblatt 91/42**

(84) Benannte Vertragsstaaten:
**AT BE CH DE ES FR GB GR IT LI LU NL SE**

(56) Entgegenhaltungen:
**EP-A- 0 037 612**
**EP-A- 0 126 722**
**DE-A- 3 015 239**

**PATENT ABSTRACTS OF JAPAN, Band 6, Nr.
90 (C-104)[968], 27. Mai 1982; & JP-A-57 22
692 (HITACHI SEISAKUSHO K.K.) 05-02-1982**

**BIOTECHNOLOGY & BIOENGINEERING, Band
27, Nr. 3, März 1985, Seiten 334-344, John
Wiley & Sons, Inc., New York, US; S.G. PAV-
LOSTATHIS et al.: "Alkaline treatment of
wheat straw for increasing anaerobic biodegradability"**

(73) Patentinhaber: **REA GESELLSCHAFT FÜR RE-
CYCLING VON ENERGIE UND ABFALL MBH
Rottmannstrasse 18
W-8000 München 2(DE)**

(72) Erfinder: **Kübler, Hans
Clemensstrasse 47
W-8000 München 40(DE)**
Erfinder: **Muck, Ottokarl
Rumfordstrasse 2
W-8000 München 5(DE)**
Erfinder: **Schmid, Martin, Dr.
Westermühlstrasse 23
W-8000 München 5(DE)**
Erfinder: **Schnell, Roland
Möckernstrasse 71
W-1000 Berlin 61(DE)**
Erfinder: **Wild, Matthias
Holzstrasse 4
W-8000 München 5(DE)**
Erfinder: **Wiljan, Harry
Hans-Sachs-Strasse 8
W-8000 München 5(DE)**

(74) Vertreter: **Reinhard, Skuhra, Weise
Friedrichstrasse 31
W-8000 München 40(DE)**

## Beschreibung

Die Erfindung bezieht sich auf ein Verfahren gemäß dem Oberbegriff des Patentanspruchs 1 sowie eine Vorrichtung nach dem Oberbegriff des Patentanspruchs 12.

Durch Einführung getrennter Sammlungen von Abfällen aus Siedlungen, Landwirtschaft und Industrie fallen Stofffraktionen an, die biotechnisch nutzbar sind.

Diese Abfälle enthalten:
- bedeutende Anteile nicht-biologischer Fremdstoffe wie Kunststoffe,Glas und Metalle;
- hohe Anteile an Papierfasern, daher z.T. aufgeschlossene Lignozellulose;
- hohe Anteile an wenig aufgeschlossenen Lignozellulosefasern;
- bedeutende Anteile an wasserextrahierbaren, biologisch verwertbaren Stoffen.

Ein hierfür typischer Abfall kann sich insbesondere wie folgt zusammensetzen:
- Fremdstoffe 20 Gew.%
- Wassergehalt 40 Gew.%
- aufgeschlossene Lignozellulose 10 Gew.%
- nichtaufgeschlossene Lignozellulose 20 Gew.%
- wasserextrahierbare, biologisch verwertbare Stoffe 5 Gew.%
- Asche in biologisch verwertbaren Materialien 5 Gew.%.

Die Aufbereitung eines derart komplexen Stoffgemisches ist im Hinblick auf eine anaerobe Vergärung der daraus gewonnenen biologisch verwertbaren Stoffe von den bestehende Verfahren nicht oder nur ansatzweise gelöst worden. Der bisher erreichte Stand der Technik kann wie folgt beschrieben werden:

Die Abtrennung der Fremdstoffe von der Rohsuspension, in der sich biotechnisch verwertbare Stoffe befinden, erfolgt gemäß DE 30 15 239 A1 nach Zerkleinerung des Rohmülls durch Mahlen,durch Beimischung von Wasser, Quellung und Flotation. Danach wird die Rohsuspension als Ganzes anaerob behandelt. Nachteilig ist bei diesem Verfahren der durch das Mahlen bedingte Eintrag von Fremdstoffteilen und Schwermetallen in die Suspension der biologisch zu verarbeitenden Stoffe, wodurch die weitere Verwertung der aus dem Verfahren abgehenden Reststoffe, z.B. als Kompost, ausgeschlossen ist. Ebenso ist die anaerobe Vergärung der erhaltenen Rohsuspension nicht umfassend, da die Anteile an aufgeschlossener und nicht aufgeschlossener Lignozellulose unverwertet bleiben. Die gleichen Nachteile treten auf, wenn,gemäß WO 82 02059, die Fremdstoffe zuerst trocken abgetrennt und dann durch Zumischen von Flüssigkeit und Naßzerkleinerung eine Rohsuspension hergestellt und diese sodann anaerob vergoren wird.

Weiterhin ist im Dokument EP B1 37 612 ein Verfahren mit einer Trockensortierung beschrieben, bei welchem der Abtrennung der Fremdstoffe nur eine geringe Bedeutung zugemessen wird. Eine verbesserte anaerobe Vergärung fester lignozellulose-haltiger Abfälle wird jedoch dadurch erreicht, daß die saure Phase des Abbaus von Zellulose/Lignozellulose von der neutralen Gasbildungsphase abgetrennt wird und die dort gebildeten Fettsäuren in der Gasbildungsphase zu Biogas umgesetzt werden.

Diese Verbesserung gilt auch für das im Dokument EP 142 873 vorgeschlagene Verfahren , bei welchem die saure Phase nicht als festes Bett, sondern als durchmischte Suspension gestaltet ist und die gebildeten Fettsäuren kontinuierlich durch Entwässerung in die Gasbildungsphase abgezogen werden.

Die beiden letztgenannten Verfahren weisen gewisse Vorteile für den biologischen Abbau der lignozellulosehaltigen Fasern auf. Nachteilig ist jedoch, daß sie keine wirksame Abtrennung der Fremdstoffe vorsehen und daß die biologische Umsetzung der wasserextrahierbarenbiologischen Stoffe zusammen mit den Faserstoffen erfolgt und damit die Wirksamkeit der anaeroben Umsetzung der Faserstoff verzögert wird. Daraus resultiert eine relativ lange Prozeßdauer.

Der Erfindung liegt die Aufgabe zugrunde, ein rasches und wirtschaftliches Verfahren und eine Vorrichtung zur Aufbereitung und anaeroben Vergärung biogenorganischer Abfälle verfügbar zu machen.

Erfindungsgemäß wird diese Aufgabe durch die im Patentanspruch 1 bzw. im Patentanspruch 12 gekennzeichneten Merkmale gelöst.

Bevorzugte Merkmale, die das Verfahren bzw. die Vorrichtung vorteilhaft weiterbilden, sind den jeweils nachgeordneten Patentansprüchen zu entnehmen.

In vorteilhafter Weise schafft die Erfindung ein Verfahren, welches die nicht-biologischen Fremdstoffe umfassend aus dem Rohabfall bzw. aus der biogen-organischen Abfallfraktion entfernt. Es wird folglich verhindert, daß Schwermetalle und bodenbelastende Substanzen aus diesen Fremdstoffen an die biologischen Abfallstoffe abgegeben werden. Die anaerobe Umsetzung der ungelösten aufgeschlossenen lignozellulosehaltigen Faserstoffe findet im Vergleich zu den sonstigen Verfahren wesentlich beschleunigt statt. Die erfindungsgemäße Vorrichtung läßt sich daher vorteilhaft kompakt konzipieren und ermöglicht in günstiger Weise eine insgesamt wirtschaftliche Verfahrensdurchführung.

In der erfindungsgemäß vorgesehenen Vorbehandlungsstufe wird die zuvor abgetrennte biogenorganische Abfallfraktion kombinierten mechani-

schen und hydraulischen Schereinwirkungen, insbesondere auf deren folienartige, fasrige organische Bestandteile ausgesetzt, und gleichzeitig werden über das zugesetzte Wasser die biologisch verwertbaren Stoffanteile aufgeweicht und mit einem großen Anteil an wasserextrahierbaren biologischen Stoffen in Lösung überführt. Die in der biogen-organischen Abfallfraktion noch verbliebenen metallischen glas- und keramikartigen Fremdstoffe werden in dieser Vorbehandlung zerkleinert, gesammelt und als Schwerstoffe gesondert abgelassen. Kunststoffe in Form von Folien und Behältern sammeln sich im Laufe der Vorbehandlung an der Oberfläche der Rohsuspension an und können dann abgeschöpft oder abgerecht werden.

Nach einer solchen Vorbehandlung erfolgt ein Austrag der Rohsuspension aus der Vorbehandlungsstufe, beispielsweise mittels einer Pumpe, und ihr Eintrag in eine sich anschließende Laugenstufe. Hier wird die Rohsuspension durch Zusatz von Chemikalien auf alkalische Bedingungen eingestellt, was gemäß einer bevorzugten Ausführungsform durch wahlweise oder kombinierten Zusatz von Natronlauge, Kalilauge, Natriumhydrogenkarbonat und/oder Kalziumhydroxid erreicht werden kann. In diesem Zustand wird die Rohsuspension auf 40 - 60°C erwärmt und für 2-12 Stunden auf einer Temperatur innerhalb dieses Bereichs gehalten.

In der sich daran anschließenden ersten Fest-Flüssig-Trennstufe erfolgt eine Auftrennung der Suspension in einen die wasserlöslichen biologischen Stoffe enthaltenden Flüssigkeitsstrom und in einen Feststoffstrom mit 20-50 Gew.-%organischer Trockenmasse. Der dabei anfallende Flüssigkeitsstrom wird dann einer Methangärung zugeführt. Dieser kann eine Vorversäuerungsstufe vorgeschaltet sein.

Der aus der Fest-Flüssig-Trennstufe erhaltene Feststoffstrom wird einer anaeroben Feststoff-Hydrolyse im sauren Bereich im meso- oder thermophilen Temperaturbereich ausgesetzt und das dabei gewonnene Hydrolysat der Methangärung zugeführt.

Die anaerobe Feststoff-Hydrolyse kann nach dem erfindungsgemäßen Verfahren auf unterschiedliche Art erfolgen. Nach einer Ausführungsvariante des Verfahrens wird der nach der ersten Fest-Flüssig-Trennstufe erhaltene Feststoffstrom nach Zustaz von Impfschlamm und Nährstoffen als Festbett unter anaeroben Bedingungen zu Methan umgesetzt.Hierbei wird von einem Feststoffstrom ausgegangen, der auf etwa 35 Gew.-% organische Trockenmasse gebracht wurde, und der mit einer nährstoffhaltigen Suspension und Kalziumkarbonat vermischt und dann im anaeroben Festbett reagieren gelassen wird. Das dabei entstehende Gasgemisch und das vergorene Restmaterial werden dann entfernt.

Gemäß einer weiteren Ausführungsvariante des erfindungsgemäßen Verfahrens wird der der Fest-Flüssigkeits-Trennstufe entnommene Feststoffstrom nach Zugabe von wässriger Flüssigkeit zumindest bis zur Rührfähigkeit, insbesondere nach Zugabe von Abwasser aus der Methangärung, einer unter kontinuierlichem oder intermittierendem Rühren stattfindenden Feststoff-Hydrolyse ausgesetzt. Aus der dabei entwickelten Gärsuspension wird in einer zweiten Fest-Flüssig-Trennstufe das gewonnene Hydrolysat abgezogen und der besagten Methangärung zugeführt. Der zweite Feststoffstrom kann nach dem Abziehen erneut in die Feststoffhydrolyse zurückgeführt werden, wobei dieser Vorgang sich so lange wiederholen läßt, bis unter den jeweils gegebenen Bedingungen keine merkliche Hydrolyse mehr stattfindet.

Eine weitere Verfahrensvariante besteht darin, daß der aus der ersten Fest-Flüssig-Trennstufe erhaltene Feststoffstrom hydrolisiert, eine Perkolation mit wässriger Flüssigkeit durchgeführt und daß Perkolat der Methangärung zugeführt wird.

Weitere vorteilhafte Einzelmerkmale des erfindungsgemäßen Verfahrens ergeben sich aus den sonstigen Patentansprüchen.

Bei der erfindungsgemäßen Vorrichtung zur Durchführung des Verfahrens ist in der Vorbehandlungsstufe eine Stofflöseeinrichtung vorgesehen, in welche jeweils eine Charge der biogen-organischen Abfallfraktion eingebracht und Wasser, beispielsweise Prozeßabwasser, insbesondere Abwasser aus der Methangärung, zugesetzt wird. Das Wasser weicht die biologischen Stoffe bis zu einem gewissen Grad auf, und die wasserextrahierbaren biologischen Stoffteile gehen in die wässrige Lösung über. Gleichzeitig entwickelt ein in der Stofflöseeinrichtung vorgesehenes Rührwerk die mechanischen und hydraulischen Scherkräfte, welche einerseits noch in der Fraktion verbliebene Fremdstoffe zerkleinern und je nachdem, ob es sich um Schweranteile oder leicht aufschwemmbare Stoffe handelt, diese einer am Bodenbereich vorgesehenen Schwerstoffaustrageinrichtung oder einer an der Oberfläche der Charge angreifenden Leichtstoffentnahmeeinrichtung zuführen. Auch erfolgt durch die kombinierte hydraulische und mechanische Schereinwirkung eine Herauslösung der mit den organischen Stoffen verbundenen Fremdstoffen, ohne eine wesentliche Zerkleinerung der gegebenen Faserbeschaffenheit, so daß auch diese Fremdstoffe von den betreffenden Austrageinrichtungen der Vorbehandlung entnommen werden können. Gemäß einer vorteilhaften Ausführungsform besteht die Leichtstoff-Entnahmeeinrichtung aus einem in die Chargenoberfläche hineinverlagerbaren Rechen, welcher die Leichtstoffe abzieht. Die schweren wasserunlöslichen Fremdstoffe, wie zerkleinertes Glas, Metall usw. werden bei der erfindungsgemäßen Vorrichtung über eine im Bodenabschnitt befindliche Schleuse bedarfsweise entfernt.

Der der Stofflöseeinrichtung nachgeschaltete Laugenreaktor ist mit einem Rührwerk und einer Heizeinrichtung versehen, so daß die eingeleitete Rohsuspension beispielsweise mit 0,5 Gew.-% Natronlauge versetzt und dann für die Dauer von etwa 3 Stunden auf ca. 50°C erwärmt im Reaktorbehälter verbleibt..

Die erste Fest-Flüssig-Trennstufe ist als mechanische Presse, insbesondere als eine Schraubenpresse ausgebildet, in der auf einfache Weise die vorbehandelte Rohsuspension in einen Flüssigkeitsstrom und einen Feststoffstrom mit etwa 20 Gew.-% Trockenrückstand aufgetrennt werden. Der Flüssigkeitsstrom wird über ein Flüssigkeitsaustrag und gegebenenfalls über eine Versäuerungsstufe dem Methanreaktor zugeleitet. Dort werden die in der Flüssigkeit gelösten Stoffe anaerob in ein methanhaltiges Gasgemisch umgewandelt.

Der Feststoffstrom wird gemäß einer bevorzugten Ausführungsform über eine Ozonierungseinrichtung oder eine mit oxidierenden Chemikalien arbeitende Einrichtung dem Hydrolysereaktor zugeführt. Der Hydrolysereaktor kann unterschiedlich aufgebaut sein und nach verschiedenen Verfahrensprinzipien arbeiten. Bei einer Ausführungsvariante mit einem Rührwerk und einer Heizung sowie einer Temperaturregelung und Einrichtungen zur Zugabe von Nährstoffen wird der ankommende Feststoffstrom mit Flüssigkeit, insbesondere Abwasser aus der Methangärung ,gemischt und eine Suspension mit etwa 10 Gew.-% Trockenmasse erzeugt, welche relativ rührfähig ist. Die Suspension wird im Reaktor bei einem pH von etwa 6,5 hydrolisiert, und das gebildete Hydrolysat wird in einer zweiten Fest-Flüssig-Trennstufe, die ebenfalls als mechanische Presse ausgebildet sein kann, chargenweise oder kontinuierlich aus der Gärsuspension herausgepreßt und dem Methanreaktor zugeleitet. Der hierbei erhaltene Feststoffstrom wird in den Hydrolysereaktor zurückgeleitet und nach einer erneuten Vermischung mit Flüssigkeit ein weiteres Mal hydrolisiert. Dieser Zyklus kann solange wiederholt werden, bis die betreffende Suspension keine praktisch verwertbaren Hydrolysierergebnisse zeigt.

Der aus der ersten Fest-Flüssig-Trennstufe erhaltene Feststoffstrom kann aber auch gemäß einer weiteren Ausführungsvariante einem Festbettreaktor zugeführt werden, in dem eine Hydrolyse und gleichzeitig eine Methangärung stattfindet.

Ferner besteht eine Ausführungsform der Erfindung darin, an den Feststoffaustrag der ersten Fest-Flüssig-Trennstufe einen Perkolationsreaktor anzuschließen, dessen Perkolataustrag mit dem erwähnten Methanreaktor in Verbindung steht.

Bei der Erprobung des erfindungsgemäßen Verfahrens und der Vorrichtung hat es sich als besondere bedeutsam erwiesen, daß für die oben beschriebene kombinierte Abtrennung der Fremdstoffe aus der biogen-organischen Müllfraktion und der materialgebundenen Fremdstoffe das Trennmedium Wasser in Verbindung mit der beschrieben Schereinwirkung beste Resultate erzielt. Zwar wird auch bereits in DE 2 406 404 als Trennmedium Wasser verwendet, jedoch nur insoweit, als Fremdstoffe von den ungelösten lignozellulosehaltigen Fasern getrennt werden sollen. Es hat sich jedoch gezeigt, daß diese Art der Abtrennung auch bei biogen-organischen Müllfraktionen der in Europa üblichen Zusammensetzung sehr gute Resultate erzielt. Vor allem hat sich es als neu und bedeutungsvoll erwiesen, daß während des Trennvorgangs im dazu verwendeten Wasser biogen-organische und anorganische Substanzen gelöst werden. Bei Versuchen mit Müllfraktionen haben sich im feststofffreien Behandlungswasser hohe organische Frachten, gemessen als chemischer Sauerstoffbedarf, ergeben. Die bei den erfindungsgemäßen Maßnahmen gezielte Einsetzung und Lenkung der kombinierten hydraulischen und mechanischen Schereinwirkung auf das biogen-organische Material beläßt die im Rohmüll gegebenenfalls noch enthaltenen Träger von Schwermetallen, z.B. Batterien, zum größten Teil unzerstört, so daß diese Müllbestandteile abgeführt werden können, was zu einer deutlichen Senkung des Schwermetallgehalts der ungelösten Fasern führt.

Ferner ist es für die Stoffauflösung von Bedeutung, daß die biogenen Faserstoffe zu einem gewissen Grad zerkleinert aber auch lateral voneinander getrennt, d.h. zerfasert werden. Gegenüber einer bislang üblichen faserverkürzenden Zerkleinerung hat dies den entscheidenden Vorteil, daß die erfindungsgemäß getrennten Fasern durch Aggregate der Fest-Flüssig-Trennung wirkungsvoller und mit relativ geringem Energieaufwand aus der erzeugten Rohsuspension abtrennbar sind.

Die Behandlung der Rohsuspension mit Alkalien und Wärme hat mehrere günstige Wirkungen: Es lösen sich ca. 50 Gew.-% mehr wasser- bzw. laugenextrahierbare biologische Stoffe in der Flüssigphase als ohne Behandlung. Ihre Umsetzung in der sauren und in der neutralen Stufe der anaeroben Vergärung ist durch das Angebot an Alkaliionen und die bessere Lösung von Bikarbonat beschleunigt. Die Abbaubarkeit der ungelösten Fasern durch den Trichoderma-viride-Zellulasekomplex wird durch diese Behandlung um das 3-5-fache erhöht. Die Behandlung mit Alkalien und Wärme verbessert die Entwässerbarkeit der Fasern deutlich. Ebenso wird die Wirksamkeit der nachfolgenden Ozonierung erhöht.

Durch die Trennung der gelaugten Suspension in gelöste und ungelöste biogene Stofffraktionen wird eine gute Anpassung an die Stoffwechselphysiologie der anaeroben Biozönose erreicht. Die gelösten biogenen Stufe, u.a. Cellobiose, Stärke, Monosaccharide, Aminosäuren, Fettsäuren, können rasch in die Zellen der Mikroorganismen transportiert werden und werden deshalb effizient umgesetzt. Würden die gelösten Stoffe zusammen mit den ungelösten Fasern vergoren, so würde der Abbau der Fasern bis zur Entfernung der gelösten Stoffe verzögert bzw. unterbleiben. Sind hingegen fast ausschließlich ungelöste Fasern vorhanden, dann stellt sich die Biozönose bei Einhaltung der übrigen Bedingungen auf den Abbau der in den Fasern enthaltenen Zellulose ein. Der aus dieser Anordnung erreichte rasche Abbau der Zellulose rechtfertigt die Auftrennung des anaeroben Prozesses.

Für die Wirksamkeit des Abbaus ungelöster Fasern ist nicht nur erforderlich, daß diese Fasern getrennt vergoren werden, sondern auch, daß die in den Fasern enthaltene Zellulose der anaeroben Biozönose in hoher Konzentration angeboten wird. Chemische Analysen der Fasern zeigen jedoch, daß bei einem größeren Anteil der Fasern die Zellulose mit Lignin verkrustet ist und deshalb für die anaeroben zelluloseabbauenden Bakterien nicht zugänglich ist. Solche Fasern müssen deshalb aufgeschlossen werden. Dies kann durch Behandlung mit Alkali und Wärme allein geschehen, doch hat es sich gezeigt, daß hierfür große Mengen an Lauge und hohe Temperaturen notwendig sind. Dies wäre sowohl unwirtschaftlich und mit den biologischen Verfahrensstufen nicht verträglich. Eine wirksame und wirtschaftliche Lösung dieses Problems findet sich in der Kombination von Alkali und Ozon in geringer Dosierung. Während die Alkalibehandlung, wie beschrieben, bereits in der Suspension günstig einsetzbar ist, bildet die Ozonierung nach der Fest-Flüssig-Trennung eine spezielle auf die Faserfraktion gerichtete Ergänzung dieser vorbereitenden Behandlung. Als Ergebnis wird der Anteil mikrobiell direkt zugänglicher Zellulose in den Fasern deutlich erhöht.

Für die optimale Wirksamkeit der anaeroben Hydrolyse von Lignozellulose-haltigen Faserstoffen ist die vorhandene Konzentration an mikrobiell direkt zugänglicher Zellulose entscheidend. Hierzu tragen gemäß der Erfindung in günstiger Weise die Faserauflösung in der Vorbehandlungsstufe, der chemisch-physikalische Aufschluß durch Alkali und Wärme, die anschließend folgende Flüssigkeitsabtrennung sowie die Ozonbehandlung des Feststoffstromes bei. Erst durch die erfindungsgemäße sinnvolle Verknüpfung dieser Verfahrensmaßnahmen kann überhaupt eine praxisgerechte Anwendung realisiert werden.

Als Ergebnis der erfindungsgemäßen Vorbehandlung als Teil des Verfahrens wird vorteilhaft die Leistung jeder Variante der anaeroben Feststoff-Hydrolyse verbessert, d. h. sowohl für die Durchführung der Feststoff-Hydrolyse mit dem einstufigen Festbettreaktor, mit dem Rührkesselreaktor als auch nach dem Prinzip Festbett mit Perkolation.

Darüber hinaus erlaubt das Verfahren, wie bereits zuvor erläutert, einen wesentlich effizienteren Abbau des Ausgangsmaterials, weil in vorteilhafter Weise die Verweilzeit der Stoffströme deutlich kürzer ist als die bei bisher bekannten Verfahren. Demzufolge nimmt auch die Ausgestaltung der erfindungsgemäßen Vorrichtung vergleichsweise deutlich weniger Platz in Anspruch.

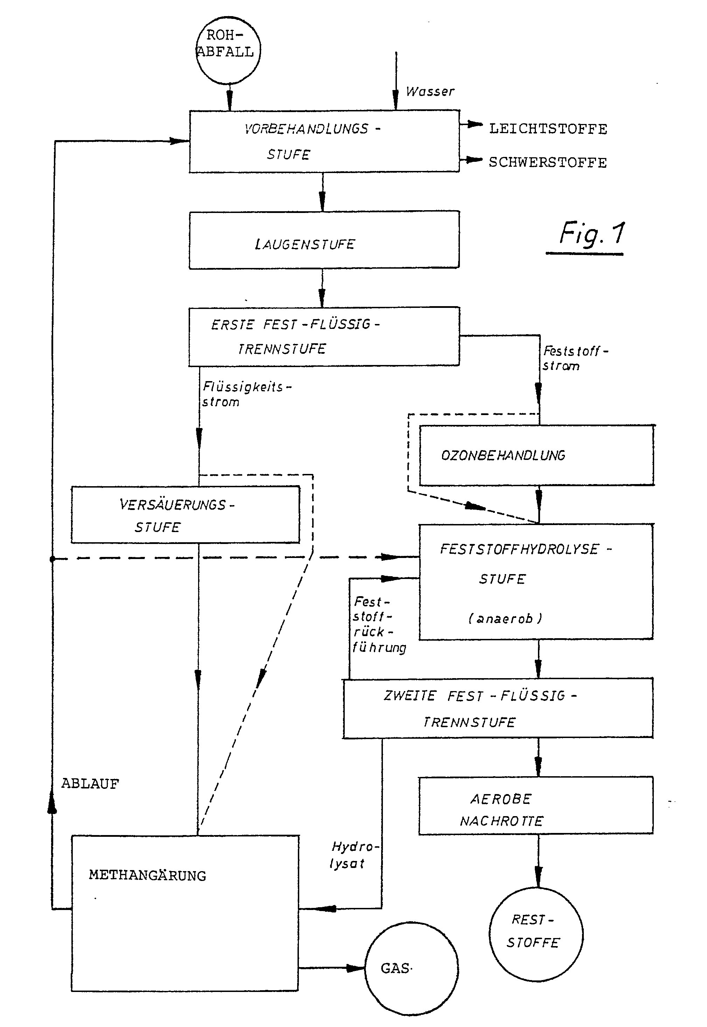

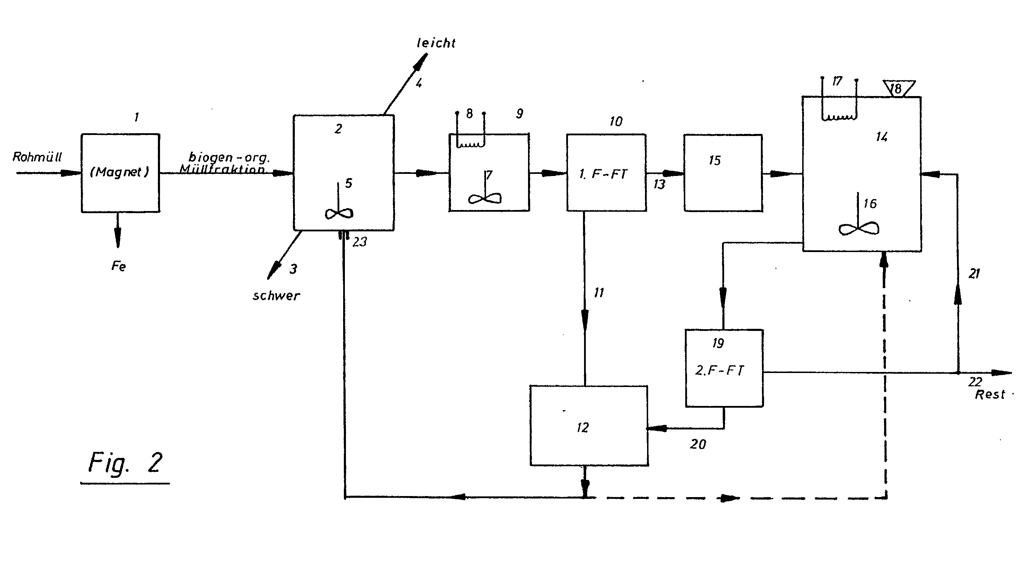

Anhand von Zeichnungen wird nachstehend in Fig. 1 das erfindungsgemäße Verfahrensprinzip und in Fig. 2 schematisch ein Vorrichtungsaufbau in Form eines Blockschaltbildes nach den Merkmalen der Erfindung beschrieben.

Einer in Fig. 1 dargestellten Vorbehandlungsstufe wird eine biogen-organische Abfallfraktion zugeführt, welche aus Rohabfall stammt, aus dem biologisch nicht verwertbare Anteile größtenteils abgesondert wurden. Außerdem erfolgt eine Einleitung von Wasser, insbesondere Abwasser aus der Methangärung, oder Flüssigabfall, in diese Vorbehandlungsstufe. Die mit Wasser versetzte biogen-organische Abfallfraktion wird in der Vorbehandlungsstufe einer kombinierten hydraulischen und mechanischen Schereinwirkung ausgesetzt, um eine Rohsuspension zu erzeugen, die von Fremdstoffen wesentlichen befreit ist. Im Anschluß an die Vorbehandlungsstufe schließt sich eine Laugenstufe an, in welche die Rohsuspension, z.B. mittels einer Pumpe, eingebracht und unter alkalischen Bedingungen bei einer Temperatur von 50-60°C für eine Dauer von 2-12 Stunden behandelt wird. Nach dieser Laugenstufe folgt eine erste Fest-Flüssig-Trennstufe, in welcher aus der laugenbehandelten Suspension ein Flüssigkeitsstrom abgezweigt wird, der die wasserlöslichen biologischen Stoffe enthält, sowie ein Feststoffstrom mit 20-50 Gew.-% organischer Trockenmasse. Der Flüssigkeitsstrom wird gemäß Fig. 1 entweder über eine Versäuerungsstufe der Methangärung zugeführt oder direkt unter Umgehung der Versäuerungsstufe der Methangärung zur Verfügung gestellt.

Der aus der ersten Fest-Flüssig-Trennstufe abgehende Feststoffstrom durchläuft gemäß Fig. 1 eine Ozonbehandlung und wird dann einer Feststoff-Hydrolysestufe zugeführt. Beim dargestellten Verfahrensprinzip ist an die Feststoff-Hydrolysestufe eine zweite Fest-Flüssig-Trennstufe angeschlossen, welche Hydrolysat vom hydrolisierten Feststoffmaterial abtrennt, dieses der Methangärung zuführt und die Feststoffe wieder der Hydrolysestufe zuleitet. Dieser Kreislauf über die zweite Fest-Flüssig-Trennstufe erfolgt so lange, bis das bearbeitete Hydrolysat keinen praktisch verwertbaren Gehalt mehr besitzt. In diesem Zustand werden die nicht weiter innerhalb des Prozesses verarbeitbaren Feststoffe einer aeroben Nachrotte zugeführt, aus der die dann noch übrigbleibenden, nicht weiterverarbeitbaren Stoffe deponiert werden.

Der in Fig. 2 gezeigte Vorrichtungsaufbau zeigt eine Vorsortieranlage 1 zur Ausscheidung biologisch nicht verwertbarer Anteile aus dem Rohabfall. In einer sich daran anschließenden Stofflöseeinrichtung 2 werden aus der biogen-organischen Abfallfraktion über eine Leichtstoffentnahmeeinrichtung 4 die Leichtstoffe und über eine Schwerstoffaustrageeinrichtung 3 die Schwerstoffe ausgeschieden.Innerhalb der Stofflöseeinrichtung 2 befindet sich ein Rührwerk 5. Ausgangsseitig ist diese Einrichtung mit einem Laugenreaktor 9 verbunden, der ein schematisch dargestelltes Rührwerk 7 sowie eine Heizeinrichtung 8 umfaßt. An diesen Laugenreaktor 9 schließt sich eine als mechanische Presse 10 ausgeführte Fest-Flüssig-Trennung an, deren Flüssigkeitsaustrag 11 an einen Methanreaktor 12 und deren Feststoffaustrag 13 mit einer Ozonierungseinrichtung 15 in Verbindung steht. Die Ozonierungseinrichtung 15 ist an einen Hydrolysereaktor 14 angeschlossen, der ebenfalls ein Rührwerk 16, eine Temperaturregeleinrichtung 17 sowie eine Einrichtung 18 zur Zugabe von Nährstoffen umfaßt. Der Hydrolysereaktor 14 ist mit einer zweiten mechanischen Presse 19 einer zweiten Fest-Flüssig-Trennung verbunden, aus der eine Hydrolysatleitung 20 in den Methanreaktor 12 hineingeht. Eine Feststoff-Rückführleitung 21 transportiert aus dieser zweiten mechanischen Presse 19 die Feststoffe in den Hydrolysereaktor 14 zurück. Sich nicht mehr zur Hydrolyse eignende Feststoffe werden über eine Austragleitung 22 einer Nachrotte bzw. einer Reststoffdeponie zugeführt. Ein Zulauf 23 an der Stofflöseeinrichtung 2 dient zur Einleitung von Prozeßabwasser in diese Einrichtung.

**Patentansprüche**

1. Verfahren zur Aufbereitung und anaeroben Vergärung biogen-organischer Abfälle, **dadurch gekennzeichnet,** daß

    a) biologisch nicht verwertbare Fraktionen aus Rohabfall abgetrennt werden,

    b) in einer Vorbehandlungsstufe die biogen-organische Abfallfraktion durch Zusatz von Wasser oder Flüssigabfall aufgeweicht und gleichzeitig einer kombinierten hydraulischen und mechanischen Schereinwirkung zur Auflösung des Abfallmaterials unter wesentlicher Beibehaltung seiner Faserbeschaffenheit ausgesetzt, und dadurch eine Rohsuspension mit 3 - 15 Gew.-% organischer Trockenmasse erzeugt wird;

    c) die Rohsuspension in einer sich anschließenden Laugenstufe durch Zusatz von Chemikalien auf alkalische Bedingungen eingestellt, auf 40 - 60 °C erwärmt und 2 bis 12 Stunden auf einer Temperatur in diesem Bereich gehalten wird;

    d) die der Laugenstufe entnommene Suspension durch eine erste Fest-Flüssig-Trennstufe in einen die wasserlöslichen biologischen Stoffe enthaltenden Flüssigkeitsstrom und in einen Feststoffstrom mit 20 - 50 Gew.-% organischer Trockenmasse aufgeteilt wird;

    e) der dadurch erhaltene Flüssigkeitsstrom dann einer Methangärung zugeführt wird; und

    f) der der ersten Fest-Flüssig-Trennstufe entnommene Feststoffstrom einer anaeroben Feststoff-Hydrolyse im sauren Bereich und im meso- oder thermophilen Temperaturbereich ausgesetzt und das dabei gewonnene Hydrolysat der besagten Methangärung zugeführt wird.

2. Verfahren nach Anspruch 1, dadurch gekennzeichnet, daß der Feststoffstrom zwischen der ersten Fest-Flüssig-Trennstufe und der Feststoff-Hydrolyse einer Behandlung entweder mit gasförmigem Ozon oder anderen oxidierenden Chemikalien ausgesetzt wird.

3. Verfahren nach Anspruch 1 oder 2, dadurch gekennzeichnet, daß der nach der ersten Fest-Flüssig-Trennstufe erhaltene Feststoffstrom nach Zusatz von Impfschlamm und Nährstoffen als Festbett unter anaeroben Bedingungen hydrolisiert und das Hydrolysat methanisiert wird.

4. Verfahren nach Anspruch 1 oder 2, dadurch gekennzeichnet, daß der der ersten Fest-Flüssig-Trennstufe entnommene Feststoffstrom nach Zugabe von Abwasser aus der Methangärung und zwar in einem Ausmaß, daß der Feststoffstrom rührfähig wird und einen Feststoffgehalt von 8 - 15 Gew.-% aufweist, einer unter Rühren stattfindenden Feststoff-Hydrolyse ausgesetzt wird, wobei das Rühren mechanisch, hydraulisch und/oder durch Gaseinpressung erfolgt und daß aus der dabei entwickelten Gärsuspension in einer zweiten Fest-Flüssigkeits-Trennstufe das gewonnene Hydrolysat abgezogen und der besagten Methangärung zugeführt wird.

5. Verfahren nach Anspruch 4, dadurch gekennzeichnet, daß der in der zweiten Fest-Flüssig-Trennstufe erhaltene zweite Feststoffstrom erneut der Feststoff-Hydrolyse zugeführt wird und daß dieser Vorgang wiederholt wird, bis unter den jeweils gegebenen Bedingungen keine merkliche Hydrolyse von Feststoffen mehr stattfindet.

6. Verfahren nach Anspruch 1 oder 2, dadurch gekennzeichnet, daß der Feststoffstrom hydrolysiert wird, daß eine Perkolation mit wäßriger Flüssigkeit durchgeführt wird, und daß das Perkolat einer Methangärung zugeführt wird.

7. Verfahren nach einem der Ansprüche 3 bis 6, dadurch gekennzeichnet, daß der nach der ersten Fest-Flüssig-Trennstufe erhaltene Flüssigkeitsstrom vor der Methangärung in einer anaeroben Versäuerungsstufe behandelt wird.

8. Verfahren nach einem der Ansprüche 4 bis 7, dadurch gekennzeichnet, daß die zweite Fest-Flüssig-Trennung zusammen mit der Feststoff-Hydrolyse entweder chargenweise und diskontinuierlich oder kontinuierlich betrieben wird.

9. Verfahren nach einem der Ansprüche 1 bis 8, dadurch gekennzeichnet, daß in der Laugenstufe wahlweise oder kombiniert Natronlauge, Kalilauge, Natriumhydrogenkarbonat und/oder Kalziumhydroxid zugegeben werden.

10. Verfahren nach einem der vorangehenden Ansprüche, dadurch gekennzeichnet, daß das Abwasser aus der Methangärung in die Vorbehandlungsstufe zurückgeführt wird.

11. Verfahren nach einem der Ansprüche 1, 2, 4 bis 10, dadurch gekennzeichnet, daß das Abwasser aus der Methangärung der Feststoffhydrolysestufe zugeführt wird.

12. Vorrichtung zur Durchführung des Verfahrens nach Anspruch 1 mit einer Vorsortieranlage (1) für Rohabfall, **dadurch gekennzeichnet,** daß die Vorbehandlungsstufe eine die biogen-organische Abfallfraktion chargenweise aufnehmende und die Rohsuspension erzeugende Stofflöseeinrichtung (2) aufweist, welche mit einer Leicht- und Schwerstoffentnahmeeinrichtung (3, 4), einem Rührwerk (5) sowie einem Zulauf (23) für Prozeßabwasser versehen ist, daß die Laugenstufe einen mit einem Rührwerk (7) und einer Heizeinrichtung (8) versehenen Laugenreaktor (9) umfaßt, und daß die erste Fest-Flüssig-Trennstufe eine mechanische Presse (10) aufweist, deren Flüssigkeitsaustrag (11) mit einem Methanreaktor (12) und deren Feststoffaustrag (13) mit einem Hydrolysereaktor (14) verbunden ist.

13. Vorrichtung nach Anspruch 12, dadurch gekennzeichnet, daß zwischen dem Feststoffaustrag (13), der mechanischen Presse (10) und dem Hydrolysereaktor (14) eine Ozonierungseinrichtung (15) vorgesehen ist und daß der Hydrolysereaktor (14) ein Rührwerk (16), eine Temperaturregelung (17) sowie Einrichtungen (18) zur Zugabe von Nährstoffen aufweist.

**Claims**

1. Process for the treatment and anaerobic digestion of biogenic waste, **characterised in that**
   a) biologically non-exploitable fractions are separated from untreated waste,
   b) in a preliminary treatment stage the biogenic waste fraction is softened by adding water or liquid waste and is simultaneously subjected to a combined hydraulic and mechanical shearing action to break up the waste material while substantially retaining its fibrous nature, thereby producing an untreated suspension having 3 - 15% by weight of organic solids;
   c) in a subsequent lye stage, the untreated suspension is adjusted to alkaline conditions by adding chemicals, is heated to 40 - 60° C and kept at a temperature within this range for 2 to 12 hours;
   d) the suspension removed from the lye stage is separated by a first solid-liquid separation stage into a liquid flow containing the water-soluble biological substances and a solids flow containing 20 - 50% by weight of organic solids;
   e) the liquid flow thereby obtained is then fed to a methane fermentation; and
   f) the solids flow removed from the first solid-liquid separation stage is subjected to anaerobic solid hydrolysis in the acid range and in the mesophilic or thermophilic temperature range and the hydrolyzate thereby obtained is fed to said methane fermentation.

2. Process according to claim 1, characterised in that between the first solid-liquid separation stage and the solid hydrolysis the solids flow is subjected to treatment either with gaseous ozone or with other oxidising chemicals.

3. Process according to claim 1 or 2, characterised in that the solids flow obtained after the first solid-liquid separation stage is hydrolysed after the addition of seeding sludge and nutrients as a fixed bed under anaerobic conditions and the hydrolyzate is methanated.

4. Process according to claim 1 or 2, characterised in that the solids flow removed from the first solid-liquid separation stage is, after the addition of effluent from the methane fermentation to such an extent that the solids flow becomes stirrable and has a solids content of 8 - 15% by weight, subjected to solid hydrolysis under agitation, with agitation being effected mechanically, hydraulically and/or by gas injection, and that, in a second solid-liquid separation stage, the hydrolyzate obtained is drawn off from the fermentation suspension thus developed and is fed to said methane fermentation.

5. Process according to claim 4, characterised in that the second solids flow obtained in the second solid-liquid separation stage is again fed to solid hydrolysis, and that said procedure is repeated until under the prevailing conditions there is no longer any noticeable hydrolysis of solids.

6. Process according to claim 1 or 2, characterised in that the solids flow is hydrolysed, that percolation with an aqueous liquid is effected, and that the percolate is fed to a methane fermentation.

7. Process according to one of claims 3 to 6, characterised in that the liquid flow obtained after the first solid-liquid separation stage is, prior to methane fermentation, treated in an anaerobic acidification stage.

8. Process according to one of claims 4 to 7, characterised in that the second solid-liquid separation together with the solid hydrolysis is effected either batchwise and discontinuously or continuously.

9. Process according to one of claims 1 to 8, characterised in that, in the lye stage, sodium hydroxide solution, potassium hydroxide solution, sodium hydrogen carbonate and/or calcium hydroxide are added selectively or in combination.

10. Process according to one of the preceding claims, characterised in that the effluent from the methane fermentation is fed back into the preliminary treatment stage.

11. Process according to one of claims 1, 2, 4 to 10, characterised in that the effluent from the methane fermentation is supplied to the solid hydrolysis stage.

12. Apparatus for effecting the process according to claim 1 having a presorting installation (1) for untreated waste,
**characterised in that**
the preliminary treatment stage has a pulping device (2) which receives batches of the biogenic waste fraction and produces the untreated suspension, said pulping device being provided with a light-solids and a heavy-solids discharging device (3, 4), an agitator (5) and a feed inlet (23) for process water, that the lye stage comprises a lye reactor (9) provided with an agitator (7) and a heating device (8), and that the first solid-liquid separation stage has a mechanical press (10), whose liquid discharge point (11) is connected to a methane reactor (12) and whose solids discharge point (13) is connected to a hydrolysis reactor (14).

13. Apparatus according to claim 12, characterised in that an ozonisation device (15) is provided between the solids discharge point (13), the mechanical press (10) and the hydrolysis reactor (14), and that the hydrolysis reactor (14) has an agitator (16), a temperature control system (17) and devices (18) for the addition of nutrients.

**Revendications**

1. Procédé de préparation et de fermentation anaérobie de déchets biogènes-organiques,
caractérisé en ce que :
a) on sépare des déchets bruts les fractions biologiquement non utilisables,
b) dans une phase de traitement préalable, on amollit la fraction de déchets biogènes-organiques en ajoutant de l'eau ou des eaux usées et on la soumet en même temps à des actions hydraulique et mécanique combinées de cisaillement en vue de désagréger la matière des déchets tout en lui conservant essentiellement son état fibreux, de façon à produire une suspension brute contenant de 3 à 15% en poids de masse organique sèche,
c) dans une phase de lavage alcalin réalisée à la suite, on ajuste la suspension brute à des conditions alcalines en ajoutant des substances chimiques, on porte sa température à 40 à 60° C et on la maintient de 2 à 12 heures à une température se trouvant dans cet intervalle de valeurs,
d) par une première phase de séparation solide-liquide, on divise la suspension provenant de la phase de lavage alcalin en un courant liquide contenant les substances biologiques solubles dans l'eau et en un courant de matières solides contenant de 20 à 50% en poids de masse organique sèche,

e) on envoie alors le courant liquide ainsi obtenu à une fermentation formant du méthane et

f) on soumet le courant de matières solides provenant de la première phase de séparation solide-liquide à une hydrolyse anaérobie des matières solides, dans le domaine acide et dans l'intervalle mésophile ou thermophile des valeurs de température, et on envoie l'hydrolysat ainsi obtenu à ladite fermentation formant du méthane.

2. Procédé suivant la revendication 1, caractérisé en ce qu'entre la première phase de séparation solide-liquide et l'hydrolyse des matières solides, on soumet le courant de matières solides à un traitement au moyen soit d'ozone gazeux, soit d'autres substances chimiques oxydantes.

3. Procédé suivant l'une des revendications 1 et 2, caractérisé en ce que le courant de matières solides obtenu à la suite de la première phase de séparation solide-liquide est hydrolysé, dans des conditions anaérobies et sous forme de lit fixe, après addition de boues d'inoculation et de substance nutritives et l'hydrolysat est transformé en méthane.

4. Procédé suivant l'une des revendications 1 et 2, caractérisé en ce qu'après addition d'eaux résiduelles provenant de la fermentation formant du méthane, ceci dans une proportion telle que le courant de matières solides provenant de la première phase de séparation solide-liquide puisse faire l'objet d'une agitation et possède une teneur en matières solides de 8 à 15% en poids, on soumet le courant de matières solides à une hydrolyse des matières solides s'effectuant sous agitation, l'agitation ayant lieu par voie mécanique, par voie hydraulique et/ou par injection de gaz sous pression, et en ce que c'est à partir de la suspension de fermentation se formant alors, que, dans une deuxième phase de séparation solide-liquide, on extrait l'hydrolysat obtenu et on l'envoie à ladite fermentation formant du méthane.

5. Procédé suivant la revendication 4, caractérisé en ce qu'on envoie à nouveau à l'hydrolyse des matières solides le second· courant de matières solides obtenu dans la seconde phase de séparation solide-liquide et en ce qu'on répète cette opération jusqu'à ce que, dans les conditions chaque fois données, il ne se produise plus d'hydrolyse notable des matières solides.

6. Procédé suivant l'une des revendications 1 et 2, caractérisé en ce qu'on hydrolyse le courant de matières solides, en ce qu'on procède à une percolation au moyen d'un liquide aqueux et en ce qu'on envoie le produit obtenu par percolation à la fermentation formant du méthane.

7. Procédé suivant l'une des revendications 3 à 6, caractérisé en ce qu'avant la fermentation formant du méthane, on traite, au cours d'une phase d'acidification anaérobie, le courant liquide obtenu à la suite de la première phase de séparation solide-liquide.

8. Procédé suivant l'une des revendications 4 à 7, caractérisé en ce qu'on exécute la seconde séparation solide-liquide, et aussi l'hydrolyse des matières solides, soit par charges et d'une manière discontinue, soit d'une manière continue.

9. Procédé suivant l'une des revendications 1 à 8, caractérisé en ce que, dans la phase de lavage alcalin, on ajoute, soit au choix, soit d'une manière combinée, de la soude caustique, de la potasse caustique, du bicarbonate de sodium et/ou de l'hydroxyde de calcium.

10. Procédé suivant l'une des revendications précédentes, caractérisé en ce qu'on renvoie à la phase de traitement préalable les eaux résiduelles provenant de la fermentation formant du méthane.

11. Procédé suivant l'une des revendications 1, 2 et 4 à 10, caractérisé en ce qu'on envoie à la phase d'hydrolyse des matières solides les eaux résiduelles provenant de la fermentation formant du méthane.

12. Installation de mise en oeuvre du procédé suivant la revendication 1, comprenant un appareillage (1) de tri préalable des déchets bruts, caractérisée en ce que l'étage de traitement préalable comprend un dispositif (2) de désagrégation des matières solides qui reçoit par charges la fraction de déchets biogènes-organiques et produit la suspension brute et qui est pourvu d'un dispositif (3, 4) d'évacuation des matières légères et des matières lourdes, d'un agitateur (5) et d'une entrée (23) prévue pour l'amenée des eaux usées du procédé, en ce que l'étage de lavage alcalin comprend une cuve de lavage alcalin (9) pourvue d'un agitateur (7) et d'un dispositif de chauffage (8) et en ce que le premier étage de séparation solide-liquide comprend une presse mécanique (10) dont la sortie de liquide (11) est reliée à une cuve de formation de méthane (12) et la sortie de matières solides (13), à une cuve d'hydrolyse (14).

13. Dispositif suivant la revendication 12, caractérisé en ce qu'il est prévu un dispositif d'ozonisation (15) entre la sortie de matières solides (13), la presse mécanique (10) et la cuve d'hydrolyse (14) et en ce que cette dernière comporte un agitateur (16), une régulation de température (17) et des dispositifs (18) d'amenée de substances nutritives.

ROH-
ABFALL

Wasser

VORBEHANDLUNGS -
STUFE

LEICHTSTOFFE

SCHWERSTOFFE

Fig. 1

LAUGENSTUFE

ERSTE FEST - FLÜSSIG -
TRENNSTUFE

Feststoff -
strom

Flüssigkeits -
strom

OZONBEHANDLUNG

VERSÄUERUNGS -
STUFE

FESTSTOFFHYDROLYSE -
STUFE

( anaerob )

Fest -
stoff -
rück -
führung

ZWEITE FEST - FLÜSSIG -
TRENNSTUFE

ABLAUF

AEROBE
NACHROTTE

Hydro -
lysat

METHANGÄRUNG

REST -
STOFFE

GAS·

11

Fig. 2